# EUROPEAN PATENT APPLICATION

(11) **EP 2 140 812 A1**
(43) Date of publication of application: **06.01.2010**
(21) Application number: 09005622.7
(22) Date of filing: 22.04.2009
(51) Int. Cl.: A61B 8/00

(54) **Ultrasonic diagnostic apparatus**

(30) Priority: 03.07.2008 KR 20080064561
(71) Applicant: MEDISON CO., LTD., Gangwon-do 250-875 (KR)
(72) Inventor: Song, Mi Ran, Seoul 130-050 (KR); Kim, Jae-Gyoung, Seoul 143-203 (KR); Song, Young Seuk, Seoul 121-260 (KR); Lee, Sun Ki, Seoul 135-080 (KR); Shin, Soo-Hwan, Seoul 137-061 (KR)
(74) Representative: Schmid, Wolfgang

(57) **Abstract**

Disclosed is an ultrasonic diagnostic device. The ultrasonic diagnostic device includes a cart, a desk formed on an upper portion of the cart, and including at least one coupling member, and a main body mounted on the desk, including at least one of a button input part and a screen display part, and engaged with the coupling member.

## Description

### BACKGROUND OF THE INVENTION

### Field of the Invention

The present invention relates to an ultrasonic diagnostic device, and more particularly, to an ultrasonic diagnostic device in which a main body thereof is readily fixed and mounted on a desk.

### Description of Related Art

In general, ultrasonic diagnostic devices are used for diagnosing diseases and other symptoms of a subject of diagnosis, and may include a probe for transmitting/receiving ultrasonic waves and a main body for controlling the ultrasonic wave transmission/reception of the probe and process received signals. Here, the main body may include a display unit for displaying transmitted/received information through the probe as images, and a button input unit for inputting diagnostic contents of the diagnosis subject.

These ultrasonic diagnostic devices may be generally fixed and mounted in a space where diagnosis is conducted due to their relatively large size and weight. Thus, in a case where the diagnosis subject has problems in his or her behaviors or a diagnosis region to be diagnosed is difficult to be diagnosed, the ultrasonic diagnostic devices that cannot be moved have problems in conducting the diagnosis.

As a result, ultrasonic diagnostic devices that can be readily carried and moved have been suggested. However, since these ultrasonic diagnostic devices are movable while not being fixed, they may be damaged due to the carelessness of a worker.

An ultrasonic diagnostic device of the present invention that may be easily detachable, carried, and moved to thereby readily conduct ultrasonic diagnosis is disclosed.

The ultrasonic diagnostic device of the present invention that may be more stably mounted on a desk is disclosed.

### SUMMARY OF THE INVENTION

An aspect of the present invention is to provide an ultrasonic diagnostic device that may be easily detachable, carried, and moved, thereby readily conducting ultrasonic diagnosis.

An aspect of the present invention is to provide an ultrasonic diagnostic device that may be more stably mounted on a desk.

According to an aspect of the present invention, there is provided an ultrasonic diagnostic device, the device including: a cart; a desk formed on an upper portion of the cart, and including at least one detachable member; and a main body mounted on the desk, including at least one of a button input part and a screen display part, and engaged with the detachable member. In this instance, the main body may be more readily and stably mounted on the desk.

Also, the detachable member may include an elastic body coupled with an edge of the main body, and the elastic body may include an uneven part engaged with the main body.

Also, the detachable member may include a rubber frame mounted on the desk, and the rubber frame may be formed on at least one side of the elastic body and adapted for supporting and fixing the elastic body.

In this instance, the rubber frame may include a first rubber frame and a second rubber frame on both sides of the elastic body. The first and second rubber frames may be elastically deformed to enable the main body to be more stably mounted on the desk when the main body is attached to and detached from the detachable member.

Also, the uneven part may be engaged with the main body. In this instance, the main body may include a protrusion and a groove used for a latching function, however, various shapes in which the main body is mounted on the desk through the latching function between the uneven part and the main body may be provided.

Also, the desk may include a front locking member formed on a front portion of the desk, and adapted for supporting and fixing a front portion of the main body. In this instance, the front locking member may include a sliding part slidably moving back and forth with respect to the main body and desk.

Also, the desk may include a slide support part forwardly extended from the desk, and the slide support part may include a slide slit adapted for guiding a slidable movement of the sliding part.

In this instance, the sliding part may further include a tightening member for fixing the sliding part to the slide support part. Specifically, the tightening member may tighten a lower portion of the slide supporting part to prevent the slide supporting part from being slidably moved any more.

### BRIEF DESCRIPTION OF THE DRAWINGS

The above and/or other aspects and advantages of the present invention will become apparent and more readily appreciated from the following detailed description, taken in conjunction with the accompanying drawings of which:
- FIG 1: is a perspective view showing an ultrasonic diagnostic device according to an exemplary embodiment of the present invention;
- FIG.2: is an exploded perspective view showing a state where a main body is mounted to and separated from a desk of the ultrasonic diagnostic device of FIG. 1;
- FIG 3: is a perspective view showing an ultrasonic diagnostic device according to another exemplary embodiment of the present invention;
- FIG. 4: is an exploded perspective view showing a state where the ultrasonic diagnostic device of FIG 3 is separated from a desk;
- FIG 5: is a perspective view showing a front locking member of the ultrasonic diagnostic device of FIG 3;
- FIG 6: is a perspective view showing an ultrasonic diagnostic device according to still another exemplary embodiment of the present invention;
- FIG. 7: is a perspective view showing a detachable member of the ultrasonic diagnostic device of FIG 6; and
- FIG 8: is a cross-sectional view showing an operation state of the detachable member of FIG 7.

### DETAILED DESCRIPTION OF EXEMPLARY EMBODIMENTS

Reference will now be made in detail to exemplary embodiments of the present invention, examples of which are illustrated in the accompanying drawings, wherein like reference numerals refer to the like elements throughout. The exemplary embodiments are described below in order to explain the present invention by referring to the figures.

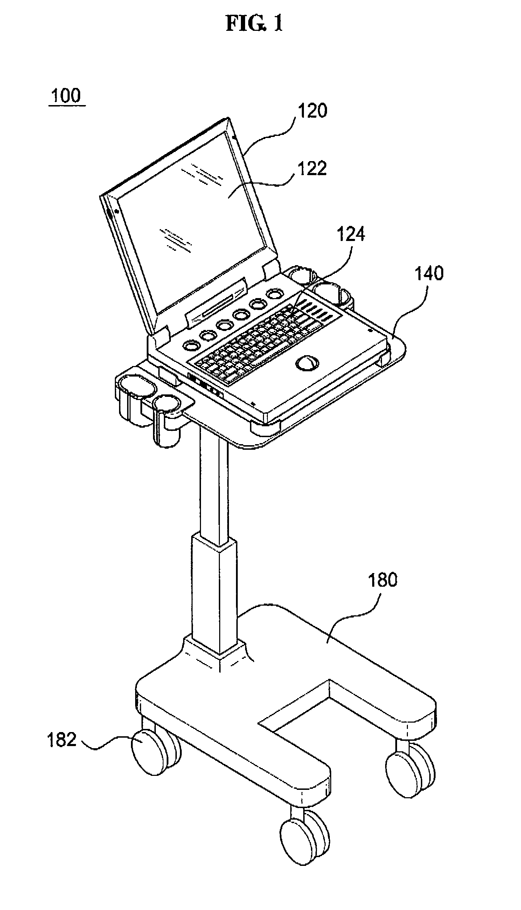

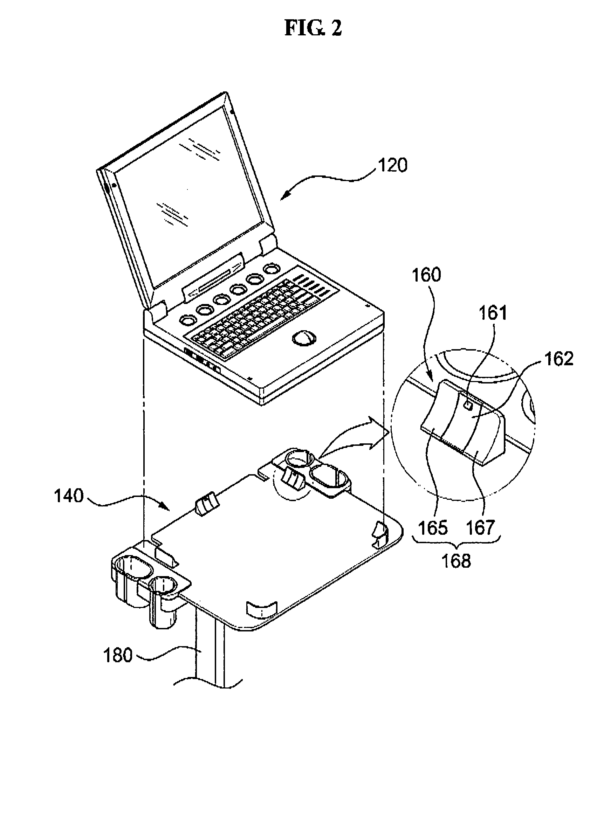

FIG. 1 is a perspective view showing an ultrasonic diagnostic device 100 according to an exemplary embodiment of the present invention and FIG. 2 is an exploded perspective view showing a state where a main body is mounted to and separated from a desk of the ultrasonic diagnostic device of FIG. 1.

Referring to FIGS. 1 and 2, the ultrasonic diagnostic device 100 according to the present exemplary embodiment may include a cart 180, a desk 140 provided on an upper portion of the cart 180 and having a certain receiving area, and a main body 120 detachably provided on the desk.

The cart 180 may include a movable roller 182 mounted on a lower portion thereof, and the desk 140 may provide a space where the ultrasonic diagnostic device 100 is seated and peripheral equipments are received. Although not shown, a probe used for ultrasonic measurement may be connected with the desk 140, and images sensed through the probe may be outputted through the main body 120.

The main body 120 may include at least one of a button input part 124 and a screen display part 122. Hereinafter, the main body 120 including both of the button input part 124 and the screen display part 122 will be described in detail. In this instance, the main body 120 may be provided with a desktop or a similar device, and also may be provided with a notebook or a similar device, as necessary. Hereinafter, for convenience of description of the ultrasonic diagnostic device 100, the main body 120 of the ultrasonic diagnostic device 100 may be described as being provided with a notebook, and a shape of the provided main body may be changed in accordance with conditions required in the present invention.

The main body 120 may be detachably mounted to the desk 140. As shown in FIG 2, the desk 140 may include at least one detachable member 160 formed thereon.

The detachable member 160 may include an elastic body 162 through which the main body 120 may be separated from and fixed on the desk 140 using a predetermined elastic force, and the elastic body 162 may include an uneven part 161 engaged with the main body 120.

In this instance, the main body 120 may be engaged with the detachable member 160, and may include a groove, protrusion, and the like formed on left/right sides or a rear side of the main body 120. The groove and protrusion may be engaged with the uneven part 161, and hereinafter, a case where the main body 120 includes the grooves formed on the left/right sides or rear side thereof to be engaged with the detachable member 160 will be described in detail.

Also, the detachable member 160 may further include a rubber frame 168. A bottom surface of the rubber frame 168 may be arranged on the desk 140, and the elastic body 162 may be provided on a side of the rubber frame 168. The rubber frame 168 may support and fix the elastic body 162 when the main body 120 and the desk 140 are elastically coupled with each other.

Also, the rubber frame 168 may restrict an elastic displacement of the elastic body 162 in which the elastic body 162 is moved while being pushed by the main body 120 when the main body 120 and the elastic body 162 are engaged with each other. Specifically, referring to FIG 2, the elastic body 162 may be separated from the rubber frame 168 by a predetermined distance. When the elastic body 162 and the rubber frame 168 are separated from each other by a predetermined distance, the elastic displacement of the elastic body 162 may be restricted while the elastic body 162 is pushed by the main body 120, or an elastic force of the elastic body 162 may be prevented from being reduced over a long period of use. Specifically, the rubber frame 168 may allow the elastic force of the elastic body 162 to be maintained while supporting a rear side of the elastic body 162.

Also, the rubber frame 168 may enable the main body 120 and the elastic body 162 to be more stably engaged with each other. Specifically, a shape of the rubber frame 168 is elastically deformed by the main body 120 when the main body 120 is engaged with the elastic body 162, and the main body 120 and the rubber frame 168 are in close contact with each other by an elastic restoring force of the rubber frame 168 after the main body 120 is mounted on the elastic body 162. In this manner, since the main body 120 may be forcibly fit in the rubber frame 168, the engagement between the main body 120 and the elastic body 162 may be more stably maintained.

As described above, so that the rubber frame 168 functions to more stably maintain the engagement between the main body 120 and the elastic body 162, the rubber frame 168 may be preferably provided on both sides of the elastic body 162. Specifically, the rubber frame 168 may include first and second rubber frames 165 and 167 formed on the both sides of the elastic body 162.

In this instance, the elastic body 162 of the detachable member 160 having a structure as described above may be embodied with a plate spring, however other springs having a lower end fixed to the desk and supporting and fixing a device by a predetermined elastic force may be provided.

Also, at least one detachable member 160 may be provided on a rear edge or left/right edges of the main body 120, however at least two detachable members 160 may be preferably provided on the rear edge or left/right of the main body 120 so as to more stably couple the main body 120 and the desk 140.

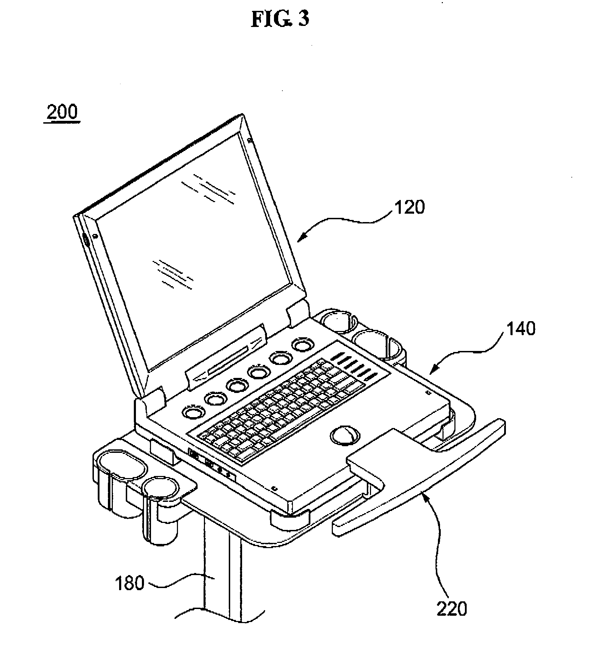

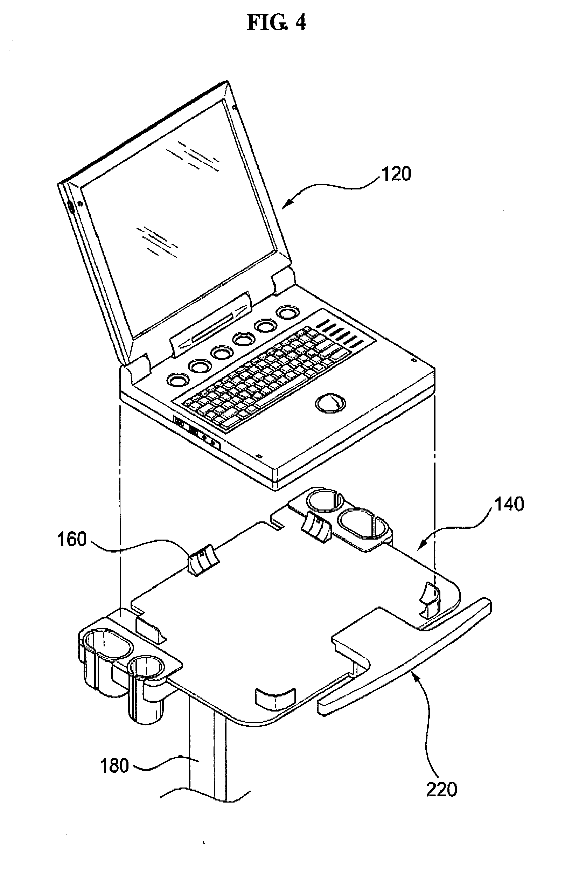

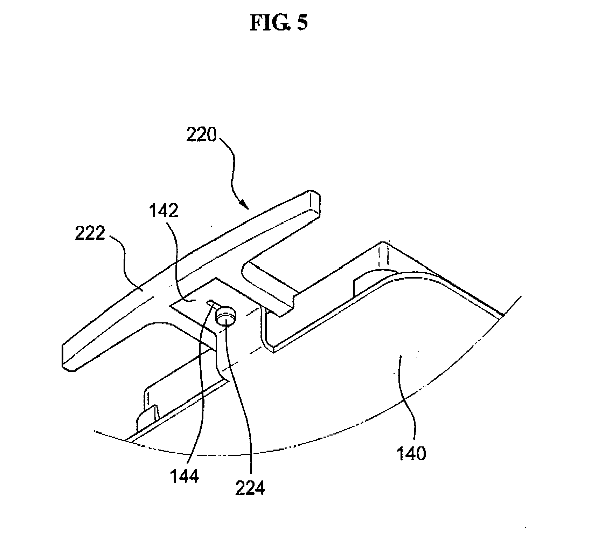

FIG. 3 is a perspective view showing an ultrasonic diagnostic device 200 according to another exemplary embodiment of the present invention, FIG. 4 is an exploded perspective view showing a state where the ultrasonic diagnostic device of FIG 3 is separated from a desk, and FIG 5 is a perspective view showing a front locking member of the ultrasonic diagnostic device of FIG. 3.

In FIGS. 3 to 5, the same reference numerals as those in FIGS. 1 and 2 may denote identical components, and differences with the ultrasonic diagnostic device of FIGS. 1 and 2 (see FIG. 1) will be hereinafter descried in detail.

Referring to FIGS. 3 to 5, the ultrasonic diagnostic device 200 according to the present exemplary embodiment may include a cart 180 (see FIG 1), a desk 140, and a main body 120. In this instance, the desk 140 may include a front locking member 220 formed on a front portion of the desk 140.

The front locking member 220 may include a sliding part 192 slidably moving back and forth with respect to the main body 120 and desk 140, and the desk may include a slide support part 142 forwardly extended from the desk 140.

The slide support part 142 may be curvedly formed into an L-shape in its profile view, and include a slide slit 144 adapted for guiding a slidable movement of the sliding part 192.

The sliding part 192 may be formed into a T-shape to allow a user to readily use the sliding part 192. Specifically, the sliding part 192 is formed into the T-shape so that the user may readily push and pull the sliding part 192 along the slide slit 144, however the sliding part 192 formed into various shapes may be provided for the convenience of the user.

Also, the sliding part 192 may be formed into a ┐-shape in its profile view, so that surfaces from a front upper surface of the main body 120 to a front edge surface thereof are supported. Specifically, the sliding part 192 may support and fix the surfaces from a front upper surface of the main body 120 to a front edge surface thereof. As a result, the main body 120 may be more stably supported and fixed on the desk 140.

Also, the slide support part 142 may include the slide slit 144 penetratedly formed in a center of the slide support part 142 along a lengthwise direction of the slide support part 142. The front locking member 220 may be located in front of the main body 120 when the sliding part 192 is slidably moved back and forth with respect to the main body 120 along the slide slit 144.

Also, the sliding part 192 may further include a tightening member 224 formed on a lower portion thereof. The tightening member 224 may be fixed in front of the main body 120 after the sliding part 192 is slidably moved. In this instance, in order to correspond to the tightening member 224, the sliding part 192 may include a receiving hole (not shown) formed on a bottom surface thereof and adapted for receiving the tightening member 224.

The tightening member 224 and the receiving hole may be provided in a similar manner of a nut and bolt configuration, and the tightening member 224 may be separated from or received in the receiving hole by a rotation thereof.

Here, the front locking member 220 may be applicable to the ultrasonic diagnostic device 100 of FIGS. 1 and 2.

Referring again to FIGS. 3 to 5, the ultrasonic diagnostic device 200 according to another exemplary embodiment may include a plurality of detachable members 160 mounted on the desk 140. In this instance, the main body 120 may further include a fastening member (not shown) engaged with the detachable member 160 and adapted for supporting and fixing the main body 120 on the desk 140.

The main body 120 having a structure as described above may be more stably supported and fixed on the desk 140. Specifically, the rear portion and left/right portions of the main body 120 may be supported and fixed on the desk 140 when the detachable member 160 and the main body 120 are coupled with each other, and the front portion of the main body 120 may be supported and fixed on the desk 140 by the front locking member 220.

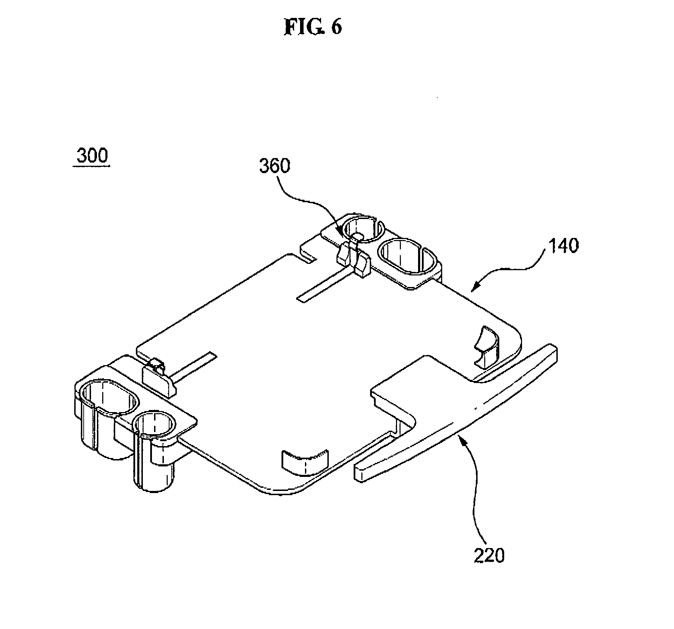

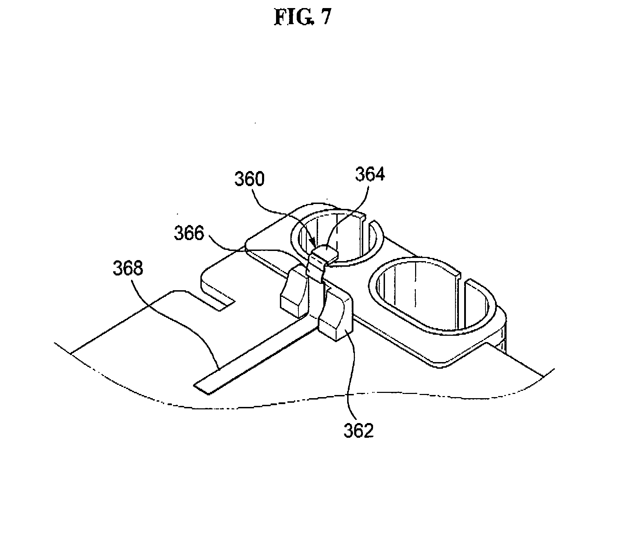

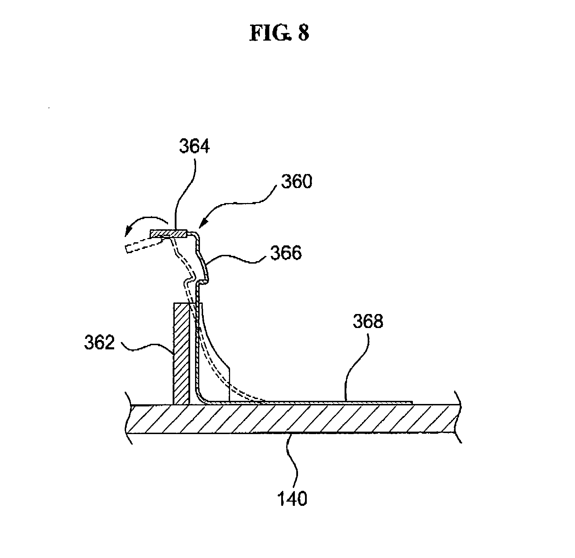

FIG 6 is a perspective view showing an ultrasonic diagnostic device 300 according to another exemplary embodiment of the present invention, FIG. 7 is a perspective view showing a detachable member of the ultrasonic diagnostic device of FIG 6, and FIG 8 is a cross-sectional view showing an operation state of the detachable member of FIG 7.

Referring to FIGS. 6 to 8, so that the ultrasonic diagnostic device 300 is mounted on the desk 140, the front locking member 220 and a detachable member 360 may be provided.

The front locking member 220 may be formed in the same or similar manner as described above to thereby obtain the same or similar effects, and thus the detachable member 360 will be hereinafter described in detail.

The detachable member 360 may include an unlock part 364 for supporting and fixing an upper surface of left/right edges of the main body 120 (see FIG. 1), and a supporting/fixing part 368 curvedly extended from the unlock part 364. Also, the supporting/fixing part 368 of the detachable member 360 may be fixed on the desk 140, and an uneven part 366 may be provided between the unlock part 364 and the supporting/fixing part 368.

Although not shown in drawings, the uneven part 366 may support and fix the left/right sides or upper surface of the main body. Specifically, the uneven part 366 may be engaged with the main body to enable the main body to be fixed on the desk 140.

As described above, a rubber frame 362 may be provided on both sides of the unlock part 364 in a similar manner as in the elastic body 162 and the rubber frame 168 shown in FIG 2, thereby more stably fixing the unlock part 364 and more stably maintain the coupling with the main body 120 (see FIG. 1). Also, the detachable member 360 may be an elastic body that is similar to the detachable member 160 shown in FIGS. 1 and 2.

Also, referring to FIG 8, the unlock part 364 may be moved inwardly or outwardly with respect to the desk when a user presses an upper surface of the unlock part 364, and the main body may be separated from or mounted to the desk in accordance with the movement of the unlock part 364. Hereinafter, an example in which the upper surface of the unlock part 364 according to the present exemplary embodiment may be elastically moved inwardly or outwardly with respect to the desk 140 when the user presses the unlock part 364 will be described in detail, and the elastic movement direction of the unlock part 364 may be changed depending on conditions, design specification, and the like required in the present invention.

The main body may be separated from the desk 140 when the unlock part 364 is elastically moved outwardly with respect to the desk. The user may again press the upper surface of the unlock part 364 when the main body is separated from the desk 140. Next, the unlock part 364 pressed again by the user may be elastically moved inwardly with respect to the desk, and the detachable member 360 elastically moved may be engaged with the left/right sides or upper surface of the main body. In this instance, the main body may be engaged with the uneven part 366 of the detachable member 360. Here, the unlock part 364 may be formed of a material such as an elastic spring that is capable of moving outwardly or inwardly with respect to the desk upon pressing the upper surface of the unlock part 364.

In this instance, the supporting/fixing part 368 may elastically support the unlock part 364 when the unlock part 364 is moved outwardly/inwardly with respect to the desk. Also, the supporting/fixing part 368 is formed relatively longer than the unlock part 364, so that a center of gravity of the unlock part 364 is located on the supporting/fixing part 368 when the unlock part 364 is moved outwardly/inwardly with respect to the desk, thereby preventing the main body from being raised or separated from the desk in accordance with the movement of the unlock part 364.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. An ultrasonic diagnostic device, the device comprising:
a cart;
a desk formed on an upper portion of the cart, and including at least one detachable member; and
a main body mounted on the desk, including at least one of a button input part and a screen display part, and engaged with the detachable member.

2. The device of claim 1, wherein the detachable member includes an elastic body coupled with an edge of the main body, and the elastic body includes an uneven part engaged with the main body.

3. The device of claim 2, wherein the detachable member includes a rubber frame mounted on the desk, and the rubber frame is formed on at least one side of the elastic body.

4. The device of claim 3, wherein the rubber frame limits an elastic displacement of the elastic body.

5. The device of claim 1, wherein the detachable member is disposed on a rear edge or left/right edges of the main body, the left/right edges of the main body corresponding to each other.

6. The device of claim 2, wherein the elastic body is a plate spring having a lower end fixed to the desk.

7. The device of claim 1, wherein the detachable member includes:
an unlock part for unlocking or restraining the engagement between the main body and the detachable member; and
a supporting/fixing part curvedly extended from the unlock part and fixed on the desk.

8. The device of claim 7, wherein an uneven part is disposed between the unlock part and the supporting/fixing part, and the uneven part supports and fixes an upper surface or left/right sides of the main body.

9. The device of claim 1, wherein the desk includes a front locking member formed on a front portion of the desk, and adapted for supporting and fixing a front portion of the main body.

10. The device of claim 9, wherein the front locking member supports and fixes the front portion of the main body, and includes a sliding part slidably moving back and forth with respect to the main body and desk.

11. The device of claim 10, wherein the desk includes a slide support part forwardly extended from the desk, and the slide support part includes a slide slit adapted for guiding a slidable movement of the sliding part.

12. The device of claim 11, wherein the sliding part further includes a tightening member for fixing the sliding part to the slide support part.
